# EUROPEAN PATENT SPECIFICATION

(11) **EP 1 657 306 B1**
(45) Date of publication and mention of the grant of the patent: **13.04.2011**
(21) Application number: 04027185.0
(22) Date of filing: 16.11.2004
(51) Int. Cl.: C12N 15/11

(54) **Gene silencing using sense DNA and antisense RNA hybrid constructs coupled to peptides facilitating the uptake into cells**
Genabschaltung (Gene silencing) durch hybride Sens-DNA und Antisens-RNA Konstrukte, gekoppelt an ein Peptid zur erleichterten Aufnahme in Zellen
Silençage genique médié au moyen d'un hybride ADN sens et ARN antisens couplé à des peptides pour faciliter leur absorption par les cellules

(43) Date of publication of application: 17.05.2006
(73) Proprietor: QIAGEN GmbH, 40724 Hilden (DE)
(72) Inventor: Weber, Martin, 42799 Leichlingen (DE); Andreou, Ioanna, 50674 Köln (DE); Bézay, Nicole, 42699 Solingen (DE)

(56) References cited:
- WO-A-01/94570
- WO-A-03/042385
- WO-A-2004/007721
- WO-A-2004/041194
- WO-A-2004/078941
- KILK K ET AL: "Targeting of antisense PNA oligomers to human galanin receptor type 1 mRNA" NEUROPEPTIDES, XX, XX, vol. 38, no. 5, October 2004 (2004-10), pages 316-324, XP004584057 ISSN: 0143-4179
- LEE L K ET AL: "Antisense technology in molecular and cellular bioengineering" CURRENT OPINION IN BIOTECHNOLOGY, LONDON, GB, vol. 14, no. 5, October 2003 (2003-10), pages 505-511, XP004568105 ISSN: 0958-1669

## Description

### BACKGROUND OF THE INVENTION

### Field of the invention:

The present invention generally relates to the field of methods for generating DNA-RNA hybrids for gene knockout transfection in vitro and in vivo. More particularly, the present invention relates to gene silencing using sense DNA (sDNA) - antisense RNA (aRNA) hybrids of 21 to 23 nucleotides in length wherein the sense DNA strand is coupled to a peptide which facilitates the uptake of the hybrid into cells. Furthermore, the present invention relates to methods for generating such sDNA-aRNA hybrids for silencing of intracellular gene expression.

### Description of the prior art:

Gene silencing or inhibiting the expression of a gene holds great therapeutic and diagnostic promise. An example of this approach is antisense technology which can be used to inhibit gene expression *in vitro* and *in vivo.* However, many problems remain with development of effective antisense technology. For example, single-stranded DNA antisense oligonucleotides exhibit only short term effectiveness and are usually toxic at the doses required for biological effectiveness. Similarly, the use of single-stranded antisense RNAs has also proved ineffective due to its fast degradation and structural instability.

Other approaches to quelling specific gene activities are posttranscriptional gene silencing (PTGS) and RNA interference (RNAi) phenomena, which have been found capable of suppressing gene activities in a variety of *in vivo* systems, including plants [Grant, Cell 96, (1999) 303-306], Drosophila melanogaster [Kennerdell and Carthew, Cell 95 (1998) 1017-1026; Misquitta. and Paterson, Proc. Natl. Acad. Sci. USA 96 (1999) 1451-1456; and Pal-Bhadra et al., J. A., Cell 99 (1999) 35-46], Caenorhabditis elegans [Tabara et al., Cell 99 (1999) 123-132, Ketting et al., Cell 99 (1999) 133-141, Fire et al., Nature 391 (1998) 806-811, Grishok et al., Science 287 (2000) 2494 -2497], zebrafish [Wargelius et al., Biochem. Biophys. Res. Commun. 263 (1999) 156-161] and mouse [Wianny et al., Nature Cell Viol. 2 (2000) 70-75]. In general, the transfection of a plasmid-like DNA structure (transgene) into cells induces PTGS phenomena, while that of a double-stranded RNA (dsRNA) causes an RNAi effect.

These phenomena appear to evoke an intracellular sequence-specific RNA degradation process, affecting all highly homologous transcripts, called cosuppression. It has been proposed that such cosuppression results from the generation of small RNA products (21-25 nucleotide bases) by an RNA-directed RNA polymerase (RdRp) [Grant supra] and/or a ribonuclease (RNase) [Ketting et al. supra, Bosher et al., Nature Cell Biology 2 (2000) 31-36, Zamore, et al., Cell 101 (2000) 25-33, and Elbashir et al., Nature 411 (2001) 494-498] activity on an aberrant RNA template, derived from the transfecting nucleic acids or viral infection.

Although an RdRp-independent endoribonucleolysis model has been proposed for the RNAi effect in Drosophila [Zamore et al. supra], the RdRp homologues were widely found in Arabidopsi thaliana as Sde-I/Sgs-2 [Yang et al., Current Biology 10 (2000) 1191], Neurospora crassa as Que-I [Cogoni et al., Nature 399 (1999) 166-169] and Caenorhabditis elegans as Ego-I [Smardon et al., Curr. Biol. 10 (2000) 169-171]. Thus, RdRp homologues appear to be a prerequisite for maintaining a long-term/inheritable PTGS/RNAi effect [Bosher et al. supra].

Although PTGS/RNAi phenomena appear to offer a potential avenue for inhibiting gene expression, they have not been demonstrated to work well in higher vertebrates and, therefore, their widespread use in higher vertebrates is still questionable. All currently found RNAi effects are based on the use of double-stranded RNA (dsRNA), which have shown to cause interferon-induced non-specific RNA degradation [Stark et al., Ayant. Rev. Biochem. 67 (1998) 227-264, and Elbashir et. al., Nature 411 (2001) 494-498 ; U. S. Pat. No. 4,289, 850 assigned to Robinson and U. S. Pat. No. 6,159, 714 assigned to Lau]. Such interferon- induced cellular response usually reduces the specific gene silencing effects of RNAi phenomena and may cause cytotoxic killing effects to the transfected cells. In mammalian cells, it has been noted that dsRNA-mediated RNAi phenomena are repressed by the interferon-induced RNA degradation when the dsRNA size is larger than 30 base-pairs or its concentrations are more than 10 nM [Elbashir supra]. For therapeutic use such as prior arts U. S. Pat. Nos. 4,945,082; 4,950652; 5,091,374 and 5,906,980 assigned to Carter, the above limitations are critical to the determination of safe dosage for drug applications. It is impossible to deliver such small size and amount of dsRNAs *in vivo* due to the high RNase activities of our bodies. Consequently, there remains a need for an effective and sustained method and composition for inhibiting gene function *in vivo* in higher vertebrates.

To increase the efficiency and stability of gene silencing effects through RNAi phenomena, messenger RNA (mRNA) -complementary DNA (cDNA) hybrids have been proposed to be one of better candidates for such purpose than dsRNAs [Grant supra, Lin et al., Nucleic Acid Res. 27 (1999) 4585-4589 and Alexeev et al., Nat. Biotechnol. 18 (2000) 43-47]. Although the mRNA-cDNA oligonucleotide has been shown to correct mutated gene expressions in mice skin and to silence oncogenes in human prostatic cancer cells [Lin et. al., Biochem. Biophys. Res. Commun. 281 (2001) 639-644], the mechanisms of these mRNA-cDNA oligonucleotides are actually based on recombinatory degradation which is not related to the RNAi phenomena [Lin et al., Current Cancer Drug Targets (2001)]. In summary, it is desirable to have a fast, stable and effective method for stimulating RNAi- related gene silencing effects, of which the results may be applied to screen special gene functions, to manipulate gene expressions *in vitro,* and even to design a therapy for genetic diseases *in vivo.*

Moreover, it has been shown that membrane permeant peptides (MPPs) are suitable candidates for the delivery of polar molecules to cells. These short amphipathic peptides have been shown to translocate across lipid bilayer in an energy independent manner somewhat similar to endocytosis [Hallbrink et al., Biochim Biophys Acta 1515 (2) (2001) 101]. In addition, these peptides can deliver large cargo molecules across membranes including nucleic acids, peptides, proteins and even paramagnetic particles up to 200 nm in diameter [Lindgren et al., Trends in Pharmacological Sci. 21 (2000) 99-103; Schwarze et al., Trends in Pharmacol. Sci. 21 (2000) 45-8 ; Josephson et al., Bioconjug. Chem. 10 (1999) 186].

In International Patent Application WO 2004/007721 a conjugate for directly targeting siRNAs to the cytoplasm of cells is disclosed, with delivery across the plasma membrane using MPPs, such as penetratin and transportan. Thiol containing siRNAs complementary to part of a luciferase transgene, or to part of a GFP transgene conjugated to MPP via a bond that is labile in the reducing cytoplasmic milieu are further disclosed. One advantage of this method is that the delivery of siRNAs conjugated to MPPs into the cytoplasm of cells, where the bond is reduced by the glutathione pool, allows the siRNAs to specifically target mRNA degradation by RNAi. However, there remains a need for an effective and sustained method and composition for inhibiting gene function.

### Description of the invention:

The present invention provides a novel composition and method for inhibiting gene function in higher eukaryotes *in vivo* and *in vitro.* Without being bound by any particular theory, this method potentially is based on an RNAi-dependent gene silencing phenomenon, which is hereafter termed sense DNA - antisense RNA (sDNA-aRNA) interference. In accordance with the present invention, peptide conjugates of sDNA-aRNA hybrids of 21 to 23 nucleotides in length are used for inhibiting gene function. The sDNA-aRNA peptide conjugates of the present invention can be used to target a gene selected from the group consisting of functional genes, pathogenic nucleic acids, viral genes/genomes, bacterial genes, mutated genes, oncogenes, etc.

Surprisingly, it has been found that sDNA-aRNA-hybrid molecules of 10 to 100, preferably 15 to 50, and most preferably 21 to 23 nucleotides in length comprising a transfection facilitating peptide - delivery peptide - coupled to the sense DNA strand has a higher efficiency in RNA interference as compared to a peptide-coupled dsRNA or a peptide-coupled sense RNA-antisense DNA-hybrid. Furthermore, the present invention allows for transfection without a transfection reagent. The application of sense DNA - antisense RNA - hybrid molecules of 21 to 23 nucleotides in length to cells in the absence of transfection reagents leads to a spontaneous uptake of the hybrid molecules by the cells.

In contrast to what is described in the prior art it has been found that only DNA/RNA molecules in which DNA is sense and RNA is antisense can be used for high level gene silencing and not as described also the opposite version (RNA sense, DNA antisense) or the DNA/DNA molecule.

Accordingly, in specific embodiments, the present invention provides a method for delivering a sense DNA and antisense RNA hybrid to a cell, in vitro the method comprising the steps of:
a) obtaining a cell
b) coupling at least one delivery peptide or peptide derivative to the sense DNA-antisense RNA hybrid or coupling in the first step the peptide with the sense DNA an then hybridizing the this first conjugate with the antisense RNA, thereby forming a peptide conjugate; and
c) contacting the cell with said peptide conjugate.

In a further embodiment the present invention provides a method for gene silencing in vitro, comprising the steps of
a) providing:
   i) a substrate expressing a targeted gene, and
   ii) a composition comprising a sDNA-aRNA hybrid peptide conjugate capable of silencing the expression of the targeted gene in the substrate;
b) treating the substrate with the composition under conditions such that gene expression in the substrate is inhibited.

The substrate can express the targeted gene *in vitro* or *in vivo.*

In a further embodiment of the present invention the peptide conjugate of the sDNA-aRNA hybrid targets a gene selected from the group consisting of functional genes, pathogenic nucleic acids, viral genes/genomes, bacterial genes, mutated genes, oncogenes.

The present invention relating to sDNA-aRNA gene knockout technology can be used as a powerful new strategy in the field of gene-based therapy. The strength of this novel strategy is in its low dose, stability, and potential long-term effects. Applications of the present invention include, without limitation, the suppression of cancer related genes, the prevention and treatment of microbe related genes, the study of candidate molecular pathways with systematic knock out of involved molecules, and the high throughput screening of gene functions based on microarray analysis, etc. The present invention can also be used as a tool for studying gene function under physiological conditions.

The DNA-RNA hybrids needed for the above purposes can be prepared by methods well known to those skilled in the art - for example by DNA solid phase synthesis via the β-cyanoethyl phosphoramidite method [Köster et al., US 4,725,677] or RNA solid phase synthesis using protected posphoramidites [Pitsch et al., US 5,986,084]. The sDNA and aRNA strands are added together in a suitable buffer - preferably in HEPES buffer - followed by denaturation for example at 90 °C for a period of time of about one minute. The annealing of both strand is performed for example at 37 °C for period of 1 hour.

Alternatively, the denaturation step can be performed in a temperature range between 90 to 95 °C over a period of time of 1 to 5 minutes.

The annealing can alternatively be achieved by cooling down the reaction mixture resulting from the denaturation step, to room temperature (20 -25 °C).

However, many other reaction conditions can be chosen from the state of the art.

The methods of the present invention further comprise the step of the conjugation of the sDNA-aRNA hybride of 21 to 23 nucleotides in length to a peptide or another substance to enhance the efficiency of transport of the RNA to living cells. These delivery peptides can include peptides known in the art for example to have cell-penetrating properties.

However, the peptides useful to carry out the present invention are not limited to this group of peptides. For example it is possible to elect the delivery peptide out of the group of so-called hydrophobic peptides with membrane anchoring function - for example signal sequence of caiman Crocodylus lg(v)light chain and hydrophobic domain HIV-gp41 [Chaolin et al., Biochemistry 36 (1997) 11179; Chaolin, Biochem. and Biophys. Res. Comm. 243 (1998) 601; Morris, Nucl. Acids Res. 25 (1997) 2730].

Alternatively transduction domain peptides or peptoides can be used - for example: and strong amphiphatic HIV TAT peptides, preferably HIV TAT - PTD peptides, SIV TAT- PTD, HSV-1-VP22, Poly-Histidine, Poly-Lysine, Poly-Ornithine, as well as Poly-Arginine (L and D - form) [Wender et al., PNAS 97 (2000) 13003; Buerger et al., JBC, 278 (2003) 37610; Vives, JBC 272 (1997) 16010; Ziegler, Biochemistry 42 (2003) 9185; Ho, Cancer Res. 61 (2001) 474; Caron, Mol. Ther. 3 (2001) 310; Schwarze, Science, 285 (1999) 1569; Eguchi, JBC 276 (2001) 26204; Futaki, Biochemistry 41 (2002) 7925] or peptide aptamers [Nagel-Wolfrum, Mol. Cancer Res. 2 (2004) 170].

Furthermore, artificial peptides being membrane permeant peptides can be used such as Transportan or Penetratin [Lindgren, Biochem. J. 2003 , Manuscript BJ 20030760; Muratovska, FEBS letters 558 (2004) 63; Derossi, Trends in Cell Biology, 1998, 84-87] or lipid interacting peptides like Antennapedia - PTD or SynB (Protegrin-derived peptides) [Drin, JBC 278 (2003) 31192; Derossi, JBC 269 (1994)10444; Astriab-Fischer, JBC 277 (2002) 22980].

In a further alternative, membrane destabilizing peptides can be chosen, for example ppTG1, ppTG20, KALA (optimized KALA: RAWA), GALA (associated with other peptides like poly lysine), MPG-peptide (HIV-gp41/ SV40 T-antigen), [Rittner, Mol. Ther. 2 (2002) 104; Morris, Nucleic Acids Res., 27 (1999) 3510; Fominaya, J. Gene Med. 2 (2000) 455] or pore forming peptides like JTS1 [Gottschalk et al., Gene Ther. 3 (1996) 448].

In another embodiment, the delivery peptide can be homeobox (hox) peptide.

Moreover, many other proteins are known to those skilled in the art being suitable to be used in the present invention [Oelke, Eur. J. Biochem. 269 (2002) 4025].

As mentioned above delivery peptides to be useful in the present invention can be, but are not limited to: TAT 47 - 57 and YGRKKRRQRRR-Cys or Cys-YGRKKRRQRRR and TAT 49 - 60 [RKKRRQRRRPPQC] [Fawell et al., TAT-Delivery of Proteins, PNAS USA, 91 (1994) 664; Vives et al., J. Biol. Chem., Vol. 272 (25) (1997) 1610; Schwarze et al., Science, 285 (1999) 1569; Nagaghara et al., Nature, 4 (12) (1998) 1449; Sihoder, Eur. J. Biochem. 269 (2002) 494] and substantially similar variants thereof, e.g., a variant is at least 65% identical thereto. Of course the percent identity can be higher, e.g., 65%, 67%, 69%, 70%, 73%, 75%, 77%, 83%, 85%, 87%, 90%, 93%, 95%, 97%, 100% identity (for example, peptides with substitutions at 1, 2, 3, 4 or more residues) [YQRKKKRRQRRRC].

In general, the substitutions are conservative substitutions. The methods of making such peptides are well known from the state of the art.

The delivery peptide can also be, but is not limited to the third α-helix of Drosophila Antennapedia homeodomain (Ant) [RQIKIWFQNRRMKWKKGGC] and substantially similar variants [Chen et al., PNAS USA 96 (1999) 4325; Astriat-Fisher et al., Pharm. Res. 19 (6) (2002) 744; Derossi, J. Biol. Chem. 269 (14) (1994) 10444] thereof; VP 22 protein from herpes simplex virus [DAATATRGRSAASRPTERPRAPARSASRPRRPRRPVE] and substantially similar variants thereof [Elliott et al., Cell 88 (1997) 223]; Nuclear localization sequence (NLS) of simian virus (SV-40) large T antigen and substantially similar variants thereof [Morris, Nucl. Acids Res. 25(14) (1997) 2730]; designed peptides (synthetic and/or chimeric cell-penetrating peptides) and variants thereof, including the Pep-1 peptide, a 21-residue peptide carrier [KETWWETWWTEWSQ-PKKKRKV] consisting of three domains: (1) a hydrophobic tryptophan-rich motif, for efficient targeting to the cell membrane; (2) NLS of SV40 large T-antigen, to improve intracellular delivery and solubility of the peptide vector; and (3) a spacer domain (SQP), containing a proline residue , to improve the flexibility and the integrity of the two hydrophobic and hydrophilic domains mentioned above, and substantially similar variants thereof [Morris et al., Nature Biotechnology, 19 (2001) 1173]; the MG/MPS delivery system a 27 residue synthetic peptide containing a hydrophobic domain derived from the fusion sequence of HIV gp41 and a hydrophilic domain derived from the nuclear localization sequence of SV 40 T-antigen [GALFLGWLGAAGSTMGAWSQPKKKRKV] and substantially similar variants thereof [Lindgren et al., Tips 21 (2000) 99; Morris, Nucl. Acids Res. 25 (14) (1997) 2730]; membrane translocating sequences (MTSs) derived from hydrophobic regions of the signal sequences from Kaposi's sarcom fibroblast growth factor 1 (K-FGF)18 and human b3 integrin 9, the fusion sequence of HIV-1 gp41; the signal sequence of the variable immunoglobulin light chain Ig(v) from Caiman crocodylus21 conjugated to NLS peptides originating from nuclear transcription factor κB (NF-κB)22, Simian virus 40 (SV40) T-antigen 23 or K-FGF; cell penetrating peptide containing 16 residues from the K-FGF MTS coupled to a F-kB NLS (ten residues) including or coupled to the SV40 T-antigen NLS (12 residues) [AAVALLPAV-LLALLAP] and variants thereof; the MTS from lg(v) light chain coupled via a peptide as sensitive linker to residues 127- 132 of SV40 T-Antigen and variants thereof, cell penetrating peptides including but not limited to penetratin, PEN (43-58 of the homeodomain of D. *melanogaster* antennapedia transcription factor, ANTP) [RQIKIWFQ-NRRMKWKK] and substantially similar variants thereof [Tung et al., Mol. Pharm. 622 (2002) 865]; signal-sequence based peptides (I) [GALFLGWLGAAGSTMGAWSQPKKKRKV] and variants thereof; signal-sequence-based peptides (II) [AAVALLPAVLLALLAP] and variants thereof; transportan [GWTLNSAGYLLKINLKALAALAKKIL] and variants thereof: Galparan, a fusion between the neuropeptide galanin-1-13 and the wasp venom peptide mastoparan and substantially similar variants thereof [Lindgren et al., Tips 21 (2000) 99; Morris, Nucl. Acids Res. 25(14) (1997) 2730]; amphiphilic modelpeptide [KLALKLALKALKAALKLA]; 18-mer amphipathic model peptide 27 [Scheller et al., J. Peptide Sci. 5 (1999) 185; Oehlke et al., Eur. J. Biochem. 269 (2002) 4025]; branched chain arginine peptides and substantially similar variants thereof [Tung et al., Bioorg. and Med. Chem. 10 (2002) 3609]; 9-polylysine protein transduction domain and substantially similar variants thereof [Park et al., Mol. Cell 13(2) (2002) 202]; β-peptides and variants thereof.

The peptides can also have modified backbones, e.g. oligocarbamate or oligourea backbones [Wang et al., J. Am. Chem. Soc. 119 (1997) 6444; Tamilarasu et al., J. Am. Chem. Soc. 121 (1999) 1597; Tamilarasu et al., Biooorg. Med. Chem. Lett., 11 (2001) 505].

Moreover, the sDNA-aRNA-hybrides can be coupled with peptoides to enhance the cellular uptake [Wender et al., PNAS 97 (2000) 13003].

As mentioned above sDNA-aRNA-hybrides can be conjugated with β-peptides [Rueping et al. Chembiochem, 02-03 (2002) 257; Umezawa et al., J. Am. Chem. Soc. 124 (2002) 368; Gademann et al., J. Med. Chem. 44 (2001) 2460].

The conjugation can be accomplished by methods well known to those skilled in the art, e.g., using the methods of Lambert et al. [Drug. Deliv. Rev. 47(1) (2001) 99 (describes nucleic acids loaded to polyalkylcyanoacrylate (PACA) nanoparticles); Fattal et al. Controlled Release 53 (1998) 137 - (describes nucleic acids bound to nanoparticles)]; Schwab et al. [Ann. Oncol., 5 Suppl. 4, (1994) 55 - (describes nucleic acids linked to intercalating agents, hydrophobic groups, polycations or PACA nanoparticles)]; and Godard et al. [Eur. J. Biochem. 232 (2) (1995) 404 - (describes nucleic acids linked to nanoparticles)].

Peptide conjugates recited herein comprise peptide portions as set forth in the sequence listing with or without cysteine residues and as disclosed in International Patent Application WO 2004/048545 pages 10 - 12 and sequences listing.

The coupling of the peptides or peptide derivatives to the aRNA or the sDNA-aRNA-hybride can be achieved via a disulfide bridge. The disulfide bridge can be built up via a so-called directed coupling reaction of a peptide thiol with a siRNA (aRNA) thiol after activation of one of the thiols with an activating agent - preferably dithio dipyridine - followed by the coupling reaction [see for example: Zeng et al., Vaccine 19 (2001) 3843].

On the other hand the coupling can be achieved via an undirected oxidation of both of the thiols using a suitable oxidation reagent. Such oxidation reagents are well known from the state of the art [Muratovska et al., FEBS 558 (2004) 63].

Moreover the conjugation can be achieved using maleic imide [Harrison et al., Nucleic Acids Res. 26 (1998) 3136; Ede et al., Bioconj. Chem. 5 (1994) 373; Mier et al., Bioconj. Chem. 11 (2000) 855].

Further alternative couplings are represented by chemoselective ligation reactions of peptides to oligonucleotides by oxime and thiazolidine formation. Both methods are known from the state of the art: the oxime conjugation can be performed by treating an oxyamine-containing peptide with an aldehyde-containing oligonucleotide or vice versa [Forget et al., Chem. Eur. J. 7 (2001) 3976].

Ligation by thiazolidine formation can be achieved by coupling a peptide, acylated with a cysteine residue, to an oligonucleotide that is derivatised by an aldehyde function [Forget et al., Chem. Eur. J. 7 (2001) 3976].

Moreover, the coupling can be achieved by a twofold reductive amination (hydro, dialkylamino-de-oxo-bisubstitution) starting from an DNA derivative having a RiboU substituent at their 3'end and being treated with sodium periodate (NalO₄) and a peptide hydrazine derivative according to method disclosed by D. Proudnikov et al. [Proudnikov et al., Nucleic Acids Res. 24 (1996) 4535].

Moreover, it is possible to achieve the coupling via the thioether or thioester method [thioether: Bonnet, J. Med. Chem. 44 (2001) 468; Thioester: Schnolzer et al., Science 256 (1992) 221].

From the state of the art many cell lines are known, which are well-established in *in vitro* transfection experiments, such as *inter alia:* Jurkat: human T-cells CH27: murine B-cells, Human PBL, Herc cells, A431: vulval carcinoma, B16: murine melanoma, U266: human myeloma, HS-68: human fibroblasts, HEK293, HeLa, SKBR3:breast carcinoma, Caco-2: human epithelial, K562, COS7, primary embryonic rat brain cells, NIH/3T3: mouse fibroblasts, C2C12: mouse myoblasts, Primary myoblasts, and fibroblasts. However, it is also possible to achieve transfection of siRNA *in vivo* using for example the TAT protein.

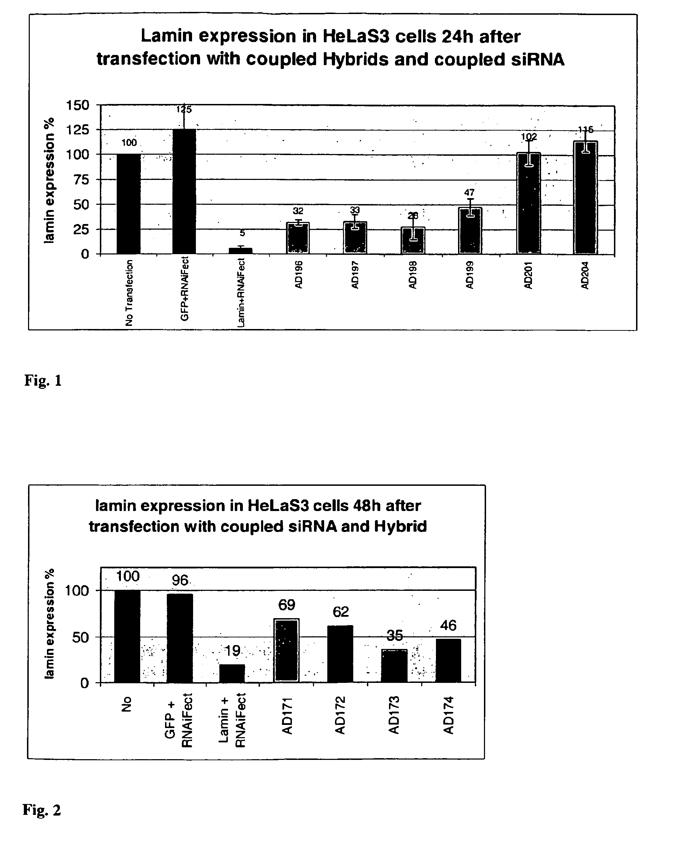

To facilitate understanding of the invention, a number of terms are defined below:
As used herein, the term "amplification" refers to nucleic acid replication involving template specificity. Template specificity is frequently described in terms of "target" specificity. Target sequences are "targets" in the sense that they are sought to be sorted out from other nucleic acids. Amplification techniques have been designed primarily for this sorting out.
As used herein, the term "antisense" refers to a nucleic acid sequence complementary to its respective mRNA molecule or a naturally occurring RNA molecule such as t-RNA, rRNA, or viral RNA. The viral RNA may be the genome of an RNA virus and may or may not encode for a functional protein. For example, the antisense RNA (aRNA) may refer to a ribonucleotide sequence complementary to a mRNA sequence, encoding for a protein, in an A-U and C-G composition, and also in the reverse orientation of the mRNA. The antisense conformation is indicated as a "-" symbol or with a "a" in front of the DNA or RNA, e.g., "aDNA" or "aRNA."
As used herein, the terms "complementary" or "complementarity" are used in reference to polynucleotides (i.e., a sequence of nucleotides) related by the base-pairing rules. For example, the sequence "A-G-T" is complementary to the sequence "T-C-A," and also to "T-C-U." Complementation can be between two DNA strands, a DNA and an RNA strand, or an RNA and another RNA strand. Complementarity may be "partial" or "complete" or "total". Partial complementarity or complementation occurs when only some of the nucleic acid bases are matched according to the base pairing rules. Complete or total complementarity or complementation occurs when the bases are completely matched between the nucleic acid strands. The degree of complementarity between nucleic acid strands has significant effects on the efficiency and strength of hybridization between nucleic acid strands. This is of particular importance in amplification reactions, as well as in detection methods which depend upon binding between nucleic acids. Percent complementarity or complementation refers to the number of mismatch bases over the total bases in one strand of the nucleic acid. Thus, a 50% complementation means that half of the bases were mismatched and half were matched. Two strands of nucleic acid can be complementary even though the two strands differ in the number of bases. In this situation, the complementation occurs between the portion of the longer strand corresponding to the bases on that strand that pairs with the bases on the shorter strand.
As used herein, the term "gene" refers to a nucleic acid (e.g., DNA) sequence that comprises coding sequences necessary for the production of a polypeptide or precursor. The polypeptide can be encoded by a full length coding sequence or by any portion of the coding sequence so long as the desired activity or functional properties (e.g., enzymatic activity, ligand binding, signal transduction, etc.) of the full-length or fragment are retained. The term "gene" encompasses both cDNA and genomic forms of a gene. A genomic form or clone of a gene contains the coding region interrupted with non-coding sequences termed "intervening regions" or "intervening sequences."
As used herein, the term "gene silencing" refers to a phenomenon whereby a function of a gene is completely or partially inhibited. Throughout the specification, the terms "silencing," "inhibition," "quelling," "knockout" and "suppression," when used with reference to gene expression or function, are used interchangeably.
As used herein, the term "homologous" or "homology" refers to a polynucleotide sequence having similarities with a mRNA sequence or naturally occurring RNA sequence such as t-RNA, rRNA or RNA genome of an RNA virus. A nucleic acid sequence may be partially or completely homologous to a particular mRNA sequence, for example. Homology may also be expressed in percentage as determined by the number of similar nucleotides over the total number of nucleotides.
As used herein, the term "sDNA" refers to a single stranded DNA that is sensehomologous to a mRNA sequence, while the term "sRNA" refers to a single stranded sense RNA that is the same as or homologous to a mRNA sequence. The term "aDNA" and "cDNA" refers to a single stranded DNA that is complementary to a mRNA sequence, while the term "aRNA" refers to a single stranded RNA that is complementary to a mRNA sequence.
As used herein, the terms "hybridize" and "hybridization" refer to the formation of complexes between nucleotide sequences which are sufficiently complementary to form complexes via Watson-Crick base pairing. Where a primer (or splice template) "hybridizes" with target (template), such complexes (or hybrids) are sufficiently stable.
As used herein, the term "nucleic acid template" refers to a double-stranded DNA molecule, double stranded RNA molecule, hybrid molecules such as DNA-RNA or RNA-DNA hybrid, or single-stranded DNA or RNA molecule.
As used herein, the term "oligonucleotide" is defined as a molecule comprised of two or more deoxyribonucleotides or ribonucleotides, preferably more than three, and usually more than ten. The exact size will depend on many factors, which in turn depends on the ultimate function or use of the oligonucleotide. The oligonucleotide may be generated in any manner, including chemical synthesis, DNA replication, reverse transcription, or a combination thereof.
As used herein, the term "primer" refers to an oligonucleotide complementary to a template. The primer complexes with the template to give a primer/template complex for initiation of synthesis by a DNA polymerase. The primer/template complex is extended by the addition of covalently bonded bases linked at its 3' end, which are complementary to the template in DNA synthesis. The result is a primer extension product. Virtually all known DNA polymerases (including reverse transcriptases) require complexing of an oligonucleotide to a single-stranded template ("priming") to initiate DNA synthesis.
As used herein, the terms "RNA-dependent DNA polymerase" and "reverse transcriptase" refer to enzymes that synthesize a complementary DNA copy from an RNA template. All known reverse transcriptases also have the ability to make a complementary DNA copy from a DNA template. Thus, reverse transcriptases are both RNA-dependent and DNA-dependent DNA polymerases. As used herein, the term "RNase H" refers to an enzyme that degrades the RNA portion of an RNA/DNA duplex. RNase H's may be endonucleases or exonucleases. Most reverse transcriptase enzymes normally contain an RNase H activity in addition to their polymerase activity. However, other sources of the RNase H are available without an associated polymerase activity. The degradation may result in separation of RNA from a RNA/DNA complex. Alternatively, the RNase H may simply cut the RNA at various locations such that portions of the RNA melt off or permit enzymes to unwind portions of the RNA.
As used herein the term 'siRNA' is intended to mean a single stranded RNA, capable for initiating RNAi or other types of RNA metabolism, and ranging from 21 to 25 - preferably from 21 to 23 nucleotides in length.
As used herein, the term "template" refers to a nucleic acid molecule being copied by a nucleic acid polymerase or a chemical synthesizer. A template can be single-stranded, double-stranded or partially double-stranded, depending on the polymerase or chemical reaction. The synthesized copy is complementary to the template, or to at least one strand of a double-stranded or partially double-stranded template. Both RNA and DNA are usually synthesized in the 5' to 3' direction (3',5'-linkages); however, some chemical synthesizers do provide the 5' to 2' phosphodiester linkages (2',5'-linkages). The 2',5'-linked and 3',5'-linked RNA/DNA share the same functional properties to the purpose of the present invention. The two strands of a nucleic acid duplex are always aligned so that the 5' ends of the two strands are at opposite ends of the duplex (and, by necessity, so then are the 3' and/or 2' ends).
As used herein, the term "transfection" refers to the introduction of foreign DNA/RNA-Hybrides into eukaryotic cells. Transfection according to the invention can be accomplished by a "membrane permeant polypetide, which represents a peptide that can translocate across cell membranes and which is bound to the DNA and/or RNA partial structure
   **Fig. 1** shows the lamin expression in HeLaS3 cells 24 h after transfection with coupled siRNA and coupled a-RNA-sDNA-hybrids according to the invention. The 'No Transfection' bar represents untreated cells. GFP und Lamin represents control transfection with uncoupled siRNA using RNAiFect. AD196-AD199 represent lamin expression in cells, which are transfected with coupled Hybrids and AD201 as well as AD204 represent lamin expression in cells transfected with coupled siRNAs, in both cases without the use of a transfection reagent. A clear silencing effect is demonstrated using coupled DNA/RNA Hybrids.
   **Fig. 2** shows the Lamin expression in HeLAS3 cells 48 h after transfection with coupled hybrids and coupled siRNA. The 'No' transfection bar represents untreated cells. 'GFP+RNAifect' and 'Lamin+RNAifect' represent control transfection with uncoupled siRNA using RNAiFect. The transfection of the hybrids AD 171 - AD 174was carried out in the presence of 2 % FCS (fetal calf serum).

### Examples

**Example 1:** Covalent coupling of peptides to siRNA or to DNA/RNA hybrids via disulfide bond formation, as an example: synthesis of a TAT-3'siRNA-conjugate (coupling at the 3'-end of the sense-strand of the siRNA duplex) and synthesis of a TAT-DNA/RNA-hybrid-conjugate (coupling at the 5'-end of the sense-strand DNA of a DNA/RNA hybrid molecule)

In order to form peptide-siRNA and peptide-DNA/RNA heterodimers and no homodimers a two step procedure through the activation of the thiol function of the peptide part (Cys) with 2,2'-dithiodipyridine was chosen:

### Step 1: Synthesis of activated HIV-TAT peptide (YGRKKRRQRRR-Cys(2-thiopyridyl)):

2,4 mg (1,45 *µ*mol, 1 eq) YGRKKRRQRRR-Cys and 1,4 mg (6,09 *µ*mol, 4,2 eq) 2,2'-dithiodipyridine are dissolved in 75 µl CH₃CN and 75 µl 0,5 M sodium acetate-buffer (pH = 4). The solution is gently agitated at room temperature. After 4 h the solution is diluted with CH₃CN/0,5 M sodium acetate to a volume of 1 ml and the reaction is analyzed by RP-HPLC (column: LUNA C18(2), 250*4,6 mm, 5*µ* (*µ*m), Phenomenex; solvent system: A: 0,1 % TFA trifluoro acetic acid in water, B: 0,1 % TFA in CH₃CN; gradient: 0-60 % B in 23 min, Rt (unactivated TAT-peptide) = 9,45 min (λ = 210 nm), Rt (activated TAT peptide) = 11,24 min (λ = 210 nm); the reaction can additionally be monitored by the formation of 2-mercaptopyridine, Rt = 8,2 min (λ = 343nm). The reaction product was purified by dialysis (MWCO = 500) at room temperature against water.

### Step 2.1: Coupling of YGRKKRRQRRR-Cys (2-thiopyridyl) to 3'-HS-(ds)siRNA through S-S-bond-formation (the HS-group is on the 3'-end of the sense-strand of the siRNA duplex):

9,83 nmol of the thiol derivative 3'-HS-(ds)siRNA (1 eq) are solubilized in 458 µl HEPES-buffer (100 mM KOAc, 30 mM HEPES-KOH, 2 mM Mg(OAc)₂) supplemented with 250mM or 500mM sodium chloride, pH 5, 34 *µ*l (49,2 nmole, 5 eq) of the above described activated TAT-solution are added and the solution is gently agitated at room temperature for 4 hours. The peptide-siRNA-conjugate is purified by dialysis (MWCO = 3500) at 4 °C against HEPES-buffer pH 7,4.

### Step 2.2: Coupling of YGRKKRRORRR-Cys (2-thiopyridyl) to 5'-HS-DNA/RNA hybrid through S-S-bond-formation (the HS-group is on the 5 '-end of the sense-strand of the DNA/RNA duplex):

9,83 nmol 5'-HS-DNA/RNA Hybrid (1 eq) are solubilized in 460 *µ*l HEPES-buffer (100 mM sodium acetate, 30 mM HEPES-KOH, 2 mM Mg(OAc)₂) supplemented with 250mM and 500mM sodium chloride, pH 5, 34 µl (49,2 nmoles, 5 eq) of the above described activated TAT-solution are added and the solution is gently agitated at room temperature for 4 hours. The peptide-siRNA-conjugate is purified by dialysis (MWCO = 3500) at 4 °C against HEPES-buffer pH 7,4.

**Example 2:** Ability of peptide coupled DNA/RNA to be taken up by the cell in the absence of transfection reagent and to induce gene silencing in comparison to peptide coupled siRNA under same conditions, efficiency comparison.

The ability of siRNA coupled with transduction peptide sequences to penetrate the cell membrane and to induce silencing of the corresponding gene without transfection reagent was tested in comparison to coupled DNA/RNA hybrids and to standardized methods for siRNA transfection using reagents based on lipid technology.

For this purpose LaminA/C siRNA coupled to HIV-TAT protein transduction domain (PTD) and DNA/RNA hybrids coupled to HIV-TAT PTD was used to perform the transfection experiments.

The peptides were coupled at the 5'site or the 3'site of the corresponding sense strand.

Coupled siRNAs/ DNA-RNA Hybrids used:
LaminA/C siRNA-3'TAT
LaminA/C DNAd(UU)s/RNAd(TT)as -5'TAT
LaminA/C DNAd(UU)s/RNAd(UU)as -5'TAT
LaminA/C target sequence CTGGACTTCCAGAAGAACA

Control siRNA transfection was performed using the following siRNAs:
LaminA/C; control for specific silencing of the LaminA/C gene (sense:
   r(CUGGACUUCCAGAAGAACA)d(TT), antisense:
   r(UGUUCUUCUGGAAGUCCAG)d(TT)
   GFP22; control for unspecific siRNA interference (sense: (5P)-
   r(GCAAGCUGACCCUGAAGUUCAU), antisense: (5P)-
   r(GAACUUCAGGGUCAGCUUGCCG)
For the transfection experiments Human cervix carcinoma HeLaS3 cells were used.

### Culture conditions prior transfection

24 h before Transfection HeLaS3 cells were seeded in a density of 6x10⁴ cells/well. They were incubated in DMEM (Dulbecco's Modification of Eagle's Medium) complemented with 10% FCS (fetal calf serum), at 37°C and 5% CO₂ atmosphere.

### Control Transfection with RNAifect

1.2 *µ*g siRNA was diluted in medium complemented with 2% FCS in a final volume of 100 µl and mixed by vortexing.

RNAifect was added in a ratio 1:6 (*µ*g RNA: µl Reagent) to the mixture, was mixed by vortexing and incubated for 15 min at room temperature.
While complex formation was taking place, the medium was aspirated from the plate and 300 µl growth medium with 2% serum was added onto the cells.
After the complex formation was complete, the complex was added drop wise onto the cells. The plate was swirled gently and cells were incubated for 24 h under normal growth conditions.

### Transfection with coupled siRNA and coupled DNA/RNA Hybrids

1.2 µg coupled siRNA or coupled DNA/RNA-hybrids were diluted in complete medium (DMEM, 2% FCS) in the final volume of 400 *µ*l and mixed by vortexing. Medium was aspirated from the plates and the 400 *µ*l siRNA/DNA:RNA hybrid suspension was added drop wise onto the cells.

The plate was swirled gently and cells were incubated for 24 h under normal growth conditions.

### Transfection efficiency analysis

24 h post transfection the medium was aspirated from the plates, the cells were lysed in 350 µl RLT buffer (3.5 M guanidinium isothiocyanate, 25 mM sodium citrate and 145 mM mercaptoethanol) and total RNA was prepared using RNeasy 96 plate.
2 µl RNA were amplified in an one tube RT-PCR using TaqMan primer and probes Amplification was performed in parallel for Lamin and GAPDH. LaminA/C expression was analyzed as quotient of the GAPDH expression. The absolute Lamin expression of the treated cells using coupled siRNA was compared to the absolute Lamin expression of the treated cells using standard siRNA and RNAifect.

### Primer and probes used in RT-PCR analysis:

| Primer: | Sequence: |
|---|---|
| LaminA_776F | GGCGGGTGGATGCTGAGAACA |
| LaminA_881R | TGTCAATCTCCACCAGTCGGG |
| hGAPDH-TM-For | GAA GGT GAA GGT CGG AGT |
| hGAPDH-TM-Rev | GAA GAT GGT GAT GGG ATT TC |

| | |
|---|---|
| TaqMan Probe | |
| LaminA_838TM | 5': FAM-ATCTACAGTGAGGAGCTGCGTGAGA-3'TAMRA |
| hGAPDH-TM | 5':FAM-CAA GCT TCC CGT TCT CAG CCT-3'TAMRA |

**Table 1**

| **Name** | **Peptide** | **Duplex** | **coupling buffer** | **nmoles Duplex : nmoles Peptide** | **dialysis in** |
|---|---|---|---|---|---|
| AD196 | Tat | Coupling with DNA(UU)/RNA(TT) | HEPES, 250mM NaCl, pH 5 | 1:5 | HEPES pH7.4 |
| AD197 | Tat | Coupling with DNA(UU)/RNA(TT) | HEPES, 500mM NaCl, pH 5 | 1:5 | HEPES pH7.4 |
| AD198 | Tat | Coupling with DNA(UU)/RNA(UU) | HEPES, 250mM NaCl, pH 5 | 1:5 | HEPES pH7.4 |
| AD199 | Tat | Coupling with DNA(UU)/RNA(UU) | HEPES, 500mM NaCl, pH 5 | 1:5 | HEPES pH7.4 |
| AD201 | Tat | Coupling with (ds)-3'-Thio-siRNA | HEPES, 250mM NaCl, pH 5 | 1:5 | HEPES pH7.4 |
| AD204 | Tat | Coupling with (ds)-3'-Thio-siRNA | HEPES, 500mM NaCl, pH 5 | 1:5 | HEPES pH7.4 |

In this set of experiments Hybrids and siRNA were treated in parallel under the same conditions for coupling with peptides. Cells were incubated with the coupled products without use of any transfection reagents. Coupled-Hybrids result in an efficient and specific silencing of the examined gene.

**Table 2**

| **Name** | **Peptide** | **Duplex** | **coupling buffer** | **nmoles Duplex: nmoles Peptide** | **dialysis in** |
|---|---|---|---|---|---|
| AD171 | Antennapedia | Coupling of (ds)-3'-Thio-siRNA | HEPES, pH 7.4 | 1:5 | HEPES pH7.4 |
| AD172 | Tat | Coupling of (ds)-3'-Thio-siRNA | HEPES, pH 7.4 | 1:5 | HEPES pH7.4 |
| AD173 | Tat | Coupling of DNA(UU)/RNA(TT)) | HEPES, pH 7.4 | 1:5 | HEPES pH7.4 |
| AD174 | Tat | Coupling of DNA(UU)/RNA(UU) | HEPES, pH 7.4 | 1:5 | HEPES pH7.4 |

Results of the transfection of coupled siRNA are shown in Figs. 1 and 2.

## Claims

1. A sense DNA-antisense-RNA-hybrid-delivery peptide conjugate of 21 to 23 nucleotides in length, wherein the delivery peptide is coupled to the sense DNA strand.

2. The conjugate of claim 1, wherein the delivery peptide is a Tat peptide

3. The conjugate of claim 1, wherein the delivery peptide is a β-peptide or a variant thereof.

4. The conjugate of claim 1, wherein the delivery peptide is a peptide derivative and has a modified backbone, preferably a oligocarbamate or oligourea backbone.

5. The conjugate of claim 1, wherein the delivery peptide is a transduction domain peptide, peptoide, or peptide aptamer.

6. The conjugate of claim 5, wherein the delivery peptide is selected from the group comprising strong amphiphatic HIV-TAT peptides, preferably HIV TAT - PTD; SIV TAT- PTD, HSV-1-VP22, Poly-Histidine, Poly-Lysine, Poly-Omithine, and Poly-Arginine (L and D-form).

7. The conjugate of claim 1, wherein the delivery peptide is an artificial peptide.

8. The conjugate of claim 7, wherein the artificial peptide is a membrane permeant peptide.

9. The conjugate of claim 8, wherein the membrane permeant peptide is Transportan or Penetratin.

10. The conjugate of claim 1, wherein the delivery peptide is a lipid interacting peptide.

11. The conjugate of claim 10, wherein the lipid interacting peptide is Antennapedia - PTD or SynB.

12. The conjugate of claim 1, wherein the delivery peptide is a membrane destabilizing peptide or a pore forming peptide.

13. The conjugate of claim 12, wherein the a membrane destabilizing peptide is ppTG1, ppTG20, KALA, RAWA, GALA, MPG-peptide (HIV-gp41/ SV40 T-antigen), or the pore forming peptide JTS1.

14. The conjugate of claim 1, wherein the delivery peptide has a membrane anchoring function.

15. The conjugate of claim 14, wherein the peptide has a signal sequence of Caiman crocodylus Ig(v)light chain or the hydrophobic domain HIV-gp41.

16. The conjugate of claim 1, wherein the delivery peptide is homeobox (hox) peptide.

17. Process for preparing a senseDNA/antisenseRNA hybrid delivery peptide conjugate according to one of claims 1 to 16, **characterized in** the following steps:
a) synthesizing an activated delivery peptide;
b) coupling at least one delivery peptide or peptide derivative to the sense DNA-antisense RNA hybrid or coupling in the first step the peptide with the sense DNA and then hybridizing the this first conjugate with the antisense RNA, thereby forming a peptide conjugate;
c) coupling the activated delivery peptide with the sDNA/aRNA-hybrid derivative to form a sDNA/aRNA hybrid delivery peptide conjugate.

18. Use of a conjugate according to one of claims 1 to 16 in an in vitro method for gene silencing.

19. A method for delivering an sDNA-aRNA-hybrid to a cell in vitro, the method comprising the steps of:
a) obtaining a cell
b) coupling at least one delivery peptide or peptide derivative to the sense DNA-antisense RNA hybrid, thereby forming a peptide conjugate according to one of claim 1 to 16; and
c) contacting the cell with said peptide conjugate.

20. The method of claim 19, wherein the cell is selected from the group comprising Jurkat: human T-cells, CH27: murine B-cells, Human PBL, Herc cells, A431: vulval carcinoma, B16: murine melanoma, U266: human myeloma, HS-68: human fibroblasts, HEK293, HeLa, SKBR3:breast carcinoma, Caco-2: human epithelial, K562, COS7, primary embryonic rat brain cells, NIH/3T3: mouse fibroblasts, C2C12: mouse myoblasts, primary myoblasts, and fibroblasts.

21. A method for gene silencingin vitro, the method comprising the steps of
a) providing
i) a substrate expressing a targeted gene,
ii) a composition comprising a sense DNA-antisense RNA hybrid delivery peptide or peptide derivative conjugate capable of silencing the expression of the targeted gene in the substrate according to one of claim 1 to 16,
b) treating the substrate with the composition under conditions such that the gene expression in the substrate is inhibited.

22. The method according to claim 21, wherein the target gene is selected from the group comprising functional genes, pathogenic nucleic acids, viral genes/genomes, bacterial genes, mutated genes, or oncogenes.

## Patentansprüche

1. Sense-DNA-Antisense-RNA-Hybrid-Zuführungspeptid-Konjugat mit einer Länge von 21 bis 23 Nukleotiden, wobei das Zuführungspeptid an den Sense-DNA-Strang gekoppelt ist.

2. Konjugat nach Anspruch 1, wobei es sich bei dem Zuführungspeptid um ein Tat-Peptid handelt.

3. Konjugat nach Anspruch 1, wobei es sich bei dem Zuführungspeptid um ein β-Peptid oder eine Variante davon handelt.

4. Konjugat nach Anspruch 1, wobei es sich bei dem Zuführungspeptid um ein Peptidderivat handelt, das ein modifiziertes Grundgerüst, vorzugsweise ein Oligocarbamat- oder Oligoharnstoff-Grundgerüst, aufweist.

5. Konjugat nach Anspruch 1, wobei es sich bei dem Zuführungspeptid um ein Transduktionsdomäne-Peptid, -Peptoid oder -Peptidaptamer handelt.

6. Konjugat nach Anspruch 5, wobei das Zuführungspeptid ausgewählt ist aus der Gruppe bestehend aus stark amphipathischen HIV-TAT-Peptiden, vorzugsweise HIV-TAT-PTD; SIV-TAT-PTD, HSV-1-VP22, Polyhistidin, Polylysin, Polyornithin und Polyarginin (L- und D-Form).

7. Konjugat nach Anspruch 1, wobei es sich bei dem Zuführungspeptid um ein künstliches Peptid handelt.

8. Konjugat nach Anspruch 7, wobei es sich bei dem künstlichen Peptid um ein membrangängiges Peptid handelt.

9. Konjugat nach Anspruch 8, wobei es sich bei dem membrangängigen Peptid um Transportan oder Penetratin handelt.

10. Konjugat nach Anspruch 1, wobei es sich bei dem Zuführungspeptid um ein Lipidwechselwirkungspeptid handelt.

11. Konjugat nach Anspruch 10, wobei es sich bei dem Lipidwechselwirkungspeptid um Antennapedia-PTD oder SynB handelt.

12. Konjugat nach Anspruch 1, wobei es sich bei dem Zuführungspeptid um ein Membrandestabilisierungspeptid oder ein Poren bildendes Peptid handelt.

13. Konjugat nach Anspruch 12, wobei es sich bei dem Membrandestabilisierungspeptid um ppTG1, ppTG20, KALA, RAWA, GALA, MPG-Peptid (HIV-gp41/SV40-T-Antigen) oder das Poren bildende Peptid JTS1 handelt.

14. Konjugat nach Anspruch 1, wobei das Zuführungspeptid eine Membranverankerungsfunktion aufweist.

15. Konjugat nach Anspruch 14, wobei das Peptid eine Signalsequenz der leichten Ig(v)-Kette aus Caiman crocodylus oder die hydrophobe Domäne HIV-gp41 aufweist.

16. Konjugat nach Anspruch 1, wobei es sich bei dem Zuführungspeptid um Homeobox-(hox-)Peptid handelt.

17. Verfahren zur Herstellung eines Sense-DNA-Antisense-RNA-Hybrid-Zuführungspeptid-Konjugats gemäß einem der Ansprüche 1 bis 16, **gekennzeichnet durch** die folgenden Schritte:
a) Synthetisieren eines aktivierten Zuführungspeptids;
b) Koppeln wenigstens eines Zuführungspeptids oder Peptidderivats an das Sense-DNA-Antisense-RNA-Hybrid oder in dem ersten Schritt Koppeln des Peptids mit der Sense-DNA und danach Hybridisieren dieses ersten Konjugats mit der Antisense-RNA, wodurch ein Peptidkonjugat gebildet wird;
c) Koppeln des aktivierten Zuführungspeptids mit dem sDNA/aRNA-Hybridderivat unter Bildung eines sDNA/aRNA-Hybrid-Zuführungspeptid-Konjugats.

18. Verwendung eines Konjugats gemäß einem der Ansprüche 1 bis 16 in einem In-vitro-Verfahren zur Genabschaltung (gene silencing).

19. Verfahren zur Zuführung eines sDNA-aRNA-Hybrids an eine Zelle in vitro, aufweisend folgende Verfahrensschritte:
a) Gewinnen einer Zelle;
b) Koppeln wenigstens eines Zuführungspeptids oder -peptidderivats an das Sense-DNA-Antisense-RNA-Hybrid, wodurch ein Peptidkonjugat gemäß einem der Ansprüche 1 bis 16 gebildet wird; und
c) Inkontaktbringen der Zelle mit dem Peptidkonjugat.

20. Verfahren nach Anspruch 19, wobei die Zelle ausgewählt ist aus der Gruppe bestehend aus Jurkat:
menschlichen T-Zellen, CH27: B-Zellen der Maus, menschlichen PBL, Herc-Zellen, A431: Vulvakarzinom, B16: Melanom der Maus, U266: menschlichem Myelom, HS-68: menschlichen Fibroblasten, HEK293, HeLa, SKBR3: Brustkarzinom, Caco-2: menschlichen Epithelzellen, K562, COS7, primären embryonalen Gehirnzellen der Ratte, NIH/3T3: Maus-Fibroblasten, C2C12: Maus-Myoblasten, primären Myoblasten und Fibroblasten.

21. Verfahren zur Genausschaltung in vitro, aufweisend folgende Verfahrensschritte:
a) Bereitstellen
i) eines Substrates, das ein Zielgen exprimiert,
ii) einer Zusammensetzung umfassend ein Sense-DNA-Antisense-RNA-Hybrid-Zuführungspeptid- oder -peptidderivat-Konjugat mit der Fähigkeit zur Ausschaltung der Expression des als Ziel vorgesehenen Gens in dem Substrat gemäß einem der Ansprüche 1 bis 16;
b) Behandeln des Substrats mit der Zusammensetzung unter Bedingungen, so dass die Genexpression in dem Substrat gehemmt wird.

22. Verfahren nach Anspruch 21, wobei das Zielgen ausgewählt ist aus der Gruppe bestehend aus funktionellen Genen, pathogenen Nukleinsäuren, viralen Genen/Genomen, bakteriellen Genen, mutierten Genen oder Onkogenen.

## Revendications

1. Conjugué d'un peptide de libération d'un hybride ADN sens-ARN antisens ayant une longueur de 21 à 23 nucléotides, dans lequel le peptide de libération est couplé au brin d'ADN sens.

2. Conjugué de la revendication 1, dans lequel le peptide de libération est un peptide Tat.

3. Conjugué de la revendication 1, dans lequel le peptide de libération est un peptide β ou un variant de ce dernier.

4. Conjugué de la revendication 1, dans lequel le peptide de libération est un dérivé peptidique et a un squelette modifié, de préférence un squelette oligocarbamate ou oligourée.

5. Conjugué de la revendication 1, dans lequel le peptide de libération est un peptide à domaine de transduction, un peptoïde ou un aptamère peptidique.

6. Conjugué de la revendication 5, dans lequel le peptide de libération est choisi dans le groupe comprenant les peptides TAT du VIH fortement amphiphatiques, de préférence le PTD TAT du VIH, le PTD TAT du SIV, le HSV-1-VP22, la poly-histidine, la polylysine, la poly-ornithine et la poly-arginine (formes L et D).

7. Conjugué de la revendication 1, dans lequel le peptide de libération est un peptide artificiel.

8. Conjugué de la revendication 7, dans lequel le peptide artificiel est un peptide de perméation de membrane.

9. Conjugué de la revendication 8, dans lequel le peptide de perméation de membrane est le transportane ou la pénétratine.

10. Conjugué de la revendication 1, dans lequel le peptide de libération est un peptide d'interaction avec les lipides.

11. Conjugué de la revendication 10, dans lequel le peptide d'interaction avec les lipides est le PTD ou le SynB d'Antennapedia.

12. Conjugué de la revendication 1, dans lequel le peptide de libération est un peptide de déstabilisation de membrane ou un peptide porogène.

13. Conjugué de la revendication 12, dans lequel le peptide de déstabilisation de membrane est le ppTG1, le ppTG20, le KALA, le RAWA, le GALA, le peptide MPG (gp41 du VIH/antigène T du SV40), ou un peptide porogène JTS1.

14. Conjugué de la revendication 1, dans lequel le peptide de libération a une fonction d'ancrage de membrane.

15. Conjugué de la revendication 14, dans lequel le peptide a une séquence signal de la chaîne légère de l'Ig(v) de Caïman croccodylus, ou le domaine hydrophobe gp41 du VIH.

16. Conjugué de la revendication 1, dans lequel le peptide de libération est un peptide homéobox (hox).

17. Procédé de préparation d'un conjugué peptide de libération d'un hybride ADN sens/ADN antisens selon l'une des revendications 1 à 16, **caractérisé par** les étapes suivantes :
a) synthèse d'un peptide de libération activé ;
b) couplage d'au moins un peptide de libération ou d'un dérivé peptidique à l'hybride ADN sens-ARN antisens, ou couplage, dans la première étape, du peptide avec l'ADN sens, puis hybridation de ce premier conjugué avec l'ARN antisens, de façon à former un conjugué peptidique ;
c) couplage du peptide de libération activé au dérivé de l'hybride ADNs/ARNa, pour former un conjugué peptide de libération de l'hybride ADNs/ARNa.

18. Utilisation d'un conjugué selon l'une des revendications 1 à 16 dans un procédé in vitro de silençage de gènes.

19. Procédé de libération d'un hybride ADNs-ARNa vers une cellule in vitro, le procédé comprenant les étapes de :
a) obtention d'une cellule,
b) couplage d'au moins un peptide de libération ou d'un dérivé peptidique à l'hybride ADN sens-ARN antisens, de façon à former un conjugué peptidique selon l'une des revendications 1 à 16 ; et
c) mise en contact de la cellule avec ledit conjugué peptidique.

20. Procédé de la revendication 19, dans lequel la cellule est choisie dans le groupe comprenant Jurkat :
cellules T humaines, CH27 : cellules B murines, PBL humain, cellules Hero, A431 : carcinome de la vulve, B16 : mélanome murin, U266 : myélome humain, HS-68 : fibroblastes humains, HEK293, HeLa, SKBR3 : cancer du sein, Caco-2 : cellules épithéliales humaines, K562, COS7 : cellules de cerveau d'embryon primaire de rat, NIH/3T3 : fibroblastes de souris, C2C12 : myoblastes de souris, myoblastes primaires et fibroblastes.

21. Procédé de silençage de gènes in vitro, le procédé comprenant les étapes de
a) mise à disposition
i) d'un substrat exprimant un gène cible,
ii) d'une composition comprenant un conjugué peptide ou dérivé peptidique de libération d'un hybride ADN sens-ARN antisens, capable de silencer l'expression du gène ciblé dans le substrat selon l'une des revendications 1 à 16,
b) traitement du substrat avec une composition dans des compositions telles que l'expression du gène dans le substrat soit inhibée.

22. Procédé selon la revendication 21, dans lequel le gène cible est choisi dans le groupe comprenant les gènes fonctionnels, les acides nucléiques pathogènes, les gènes/génomes viraux, les gènes bactériens, les gènes mutés et les oncogènes.
